# EUROPÄISCHE PATENTSCHRIFT

(11) **EP 1 536 971 B1**
(45) Veröffentlichungstag und Bekanntmachung des Hinweises auf die Patenterteilung: **13.06.2007**
(21) Anmeldenummer: 03793749.7
(22) Anmeldetag: 22.08.2003
(51) Int. Cl.: B60K 28/06, A61B 5/18

(54) **VERFAHREN UND VORRICHTUNG ZUR ERKENNUNG DES AUFMERKSAMKEITSGRADES EINES FAHRZEUGFÜHRERS**
METHOD AND DEVICE FOR RECOGNIZING THE LEVEL OF AWARENESS OF A VEHICLE DRIVER
PROCEDE ET DISPOSITIF D'IDENTIFICATION DU DEGRE D'ATTENTION D'UN CONDUCTEUR DE VEHICULE

(30) Priorität: 04.09.2002 DE 10241624
(43) Veröffentlichungstag der Anmeldung: 08.06.2005
(73) Patentinhaber: Volkswagen Aktiengesellschaft, 38436 Wolfsburg (DE)
(72) Erfinder: BROSIG, Stefan, 29386 Hankensbüttel (DE); APEL, Andreas, 38228 Salzgitter (DE)
(74) Vertreter: Schneider, Henry
(86) Internationale Anmeldenummer: PCT/EP2003/009357
(87) Internationale Veröffentlichungsnummer: WO 2004/022376

(56) Entgegenhaltungen:
- WO-A-02/17787
- DE-A- 4 400 207
- US-A- 5 745 031
- US-A- 5 769 085
- PATENT ABSTRACTS OF JAPAN vol. 1999, no. 14, 22. Dezember 1999 (1999-12-22) -& JP 11 263143 A (YAZAKI CORP), 28. September 1999 (1999-09-28)
- PATENT ABSTRACTS OF JAPAN vol. 1996, no. 12, 26. Dezember 1996 (1996-12-26) -& JP 08 202998 A (ISUZU MOTORS LTD), 9. August 1996 (1996-08-09)
- PATENT ABSTRACTS OF JAPAN vol. 2000, no. 03, 30. März 2000 (2000-03-30) -& JP 11 339199 A (TOYOTA MOTOR CORP), 10. Dezember 1999 (1999-12-10)
- PATENT ABSTRACTS OF JAPAN vol. 018, no. 025 (M-1542), 14. Januar 1994 (1994-01-14) -& JP 05 262162 A (NISSAN MOTOR CO LTD), 12. Oktober 1993 (1993-10-12)
- PATENT ABSTRACTS OF JAPAN vol. 2000, no. 10, 17. November 2000 (2000-11-17) -& JP 2000 193545 A (ISUZU MOTORS LTD), 14. Juli 2000 (2000-07-14)

## Beschreibung

Die Erfindung betrifft ein Verfahren und eine Vorrichtung zur Erkennung des Aufmerksamkeitsgrades eines Fahrzeugführers, wobei ein Lenkverhalten des Fahrzeugführers überwacht wird, mit den in den Oberbegriffen der Ansprüche 1, 2 und 11, 12 genannten Merkmalen.

Es ist allgemein bekannt, dass bei Führern von Fahrzeugen besonders hohe Anforderungen an deren Aufmerksamkeit (Vigilanz) gestellt werden. Eine Verringerung der Aufmerksamkeit des Führers des Fahrzeuges, insbesondere durch Müdigkeit und/oder durch eine Monotonie der momentanen Fahrsituation, birgt ein erhöhtes Unfallrisiko in sich.

Verfahren und Vorrichtungen zur Verbesserung der Aufmerksamkeit eines Fahrzeugführers sind bekannt. So offenbart beispielsweise die DE 44 00 207 C2 eine Vorrichtung und ein Verfahren, bei dem eine Herzschlaginformation des Fahrzeugführers mit einer Lenkinformation verknüpft wird. Hierbei wird mittels eines Lenkwinkelsensors die Betätigung eines Lenkrades durch einen Fahrzeugführer detektiert. So soll die Betätigung des Lenkrades durch den Fahrzeugführer überwacht werden, indem spezifische Lenkperioden vor einem Rechts- beziehungsweise Linksdrehen des Lenkrades durch den Fahrzeugführer überwacht werden. Hier liegt der Ansatz zugrunde, dass - je größer die Aufmerksamkeit des Fahrzeugführers ist - so genannte Feinlenkperioden auftreten, die der Kurshaltung des Fahrzeuges dienen. Treten diese Feinlenkperioden nicht oder nur in größeren Zeitabständen auf, spricht dies für eine nachlassende Aufmerksamkeit des Fahrzeugführers.

Allgemein ist bekannt, dass eine Lenkbewegung eines Fahrzeuges entweder aufgrund eines Fahrerwunsches oder durch äußere Einflüsse, beispielsweise böiger Wind, schlechte Straßenverhältnisse oder dergleichen, die ein Gegenlenken durch den Fahrzeugführer erfordern, auftreten kann.

Ferner ist allgemein bekannt, Fahrzeuge mit elektronischem Stabilitätssystem (ESP) und/oder elektrischer Lenkunterstützung (EPS) auszustatten. Derartige Systeme unterstützen den Fahrzeugführer beim Führen des Fahrzeuges, indem Regelsysteme Abweichungen zwischen Soll-Verhalten und Ist-Verhalten einzelner Komponenten erfassen und entsprechende Gegenmaßnahmen selbsttätig einleiten.

Die JP11-263143 offenbart ein Verfahren zur Erkennung des Aufmerksamkeitsgrades eines Fahrzeugführers bzw. eine dazu korrespondierende Vorrichtung, wobei das Lenkverhalten des Fahrzeugführers überwacht wird und ein Phasenverlauf zwischen einer Änderung eines Lenkwinkels eines lenkbaren Rades des Fahrzeuges und einer Änderung des Lenkradwinkels dadurch ausgewertet wird, dass die Länge einer Zeitspanne zwischen einer Änderung des Lenkwinkels und einer darauf folgenden Änderung des Lenkradwinkels durch den Fahrer ausgewertet wird.

Der Erfindung liegt die Aufgabe zugrunde, ein Verfahren und eine Vorrichtung der gattungsgemäßen Art zu schaffen, mittels denen in einfacher Weise die Erkennung des Aufmerksamkeitsgrades eines Fahrzeugführers möglich ist.

Erfindungsgemäß wird diese Aufgabe durch ein Verfahren mit den in den Ansprüchen 1 und 2 genannten Merkmalen gelöst. Dadurch, dass ein Phasenverlauf zwischen einer Änderung eines Lenkwinkels.wenigstens eines lenkbaren Rades des Fahrzeuges und einer Änderung eines Lenkradwinkels ausgewertet wird, wird in besonders einfacher und zuverlässiger Weise der Aufmerksamkeitsgrad eines Fahrzeugführers bestimmbar. Bei dem erfindungsgemäßen Verfahren wird der Umstand ausgenutzt, dass bei einer äußeren Lenkbeeinflussung des Fahrzeuges der Fahrzeugführer eine Gegenlenkbewegung als Korrektur seines Lenkwunsches veranlassen muss. Die äußere Lenkbeeinflussung führt zu einer Veränderung des Lenkwinkels wenigstens eines lenkbaren Rades des Fahrzeuges, nämlich der Ist-Lenkwinkel weicht dann vom Soll-Lenkwinkel ab. Das Gegenlenken des Fahrzeugführers erfolgt über das Lenkrad, so dass die Lenkbewegung des Fahrzeugführers durch eine Veränderung des Lenkradwinkels erfassbar ist. Aufgrund unvermeidlicher Reaktionszeiten kommt es hierbei zu einem Unterschied des Phasenverlaufes zwischen dem Lenkwinkel des wenigstens einen lenkbaren Rades und dem Lenkradwinkel. Dieser Unterschied zwischen Phasenverlauf des Lenkwinkels und des Lenkradwinkels ist somit ein Maß für die Reaktionsfähigkeit (Reaktionsschnelligkeit) des Fahrzeugführers, so dass über eine nachlassende Reaktionsfähigkeit auf einen nachlassenden Aufmerksamkeitsgrad des Fahrzeugführers in einfacher Weise geschlossen werden kann.

Für die Erkennung des Aufmerksamkeitsgrades eines Fahrzeugführers ist vorgesehen, dass der Phasenverlauf in Zeitspannen ausgewertet wird, in denen Lenkbewegungen des Fahrzeuges nicht von einem Lenkungswunsch des Fahrzeugführers ausgehen. Hierdurch wird der Aufwand zur Erkennung des Aufmerksamkeitsgrades des Fahrzeugführers wesentlich verringert. Nämlich erst dann, wenn eine Lenkbewegung des Fahrzeuges durch äußere Lenkungseinflüsse hervorgerufen ist, setzt das erfindungsgemäße Verfahren zur Erkennung des Aufmerksamkeitsgrades ein. In Zeitspannen, in denen eine Lenkungsbewegung entweder durch einen Lenkungswunsch des Fahrzeugführers oder keine Lenkungsbeeinflussung erfolgt, setzt das erfindungsgemäße Verfahren vorzugsweise aus.

Deshalb wird der Phasenverlauf innerhalb einer Zeitspanne ausgewertet, in der eine Änderung des Lenkradwinkels einer Änderung des Lenkwinkels folgt. Die Erkennung des Aufmerksamkeitsgrades des Fahrzeugführers erfolgt gemäß Anspruch 1 durch Auswertung des Gradienten des Lenkradwinkels (α) in einer Zeitspanne (t₂ bis t₃), wobei t₂ der Zeitpunkt ist, zu dem eine Änderung des Lenkradwinkels (α) erfolgt und t₃ der Zeitpunkt ist, ab dem der Fahrzeugführer durch Betätigung des Lenkrades beginnt, einen Ausgleich herbeizuführen. Gemäß Anspruch 2 ist es vorgesehen, den Gradienten des Lenkradwinkels (α) mit dem Gradienten des Lenkwinkels (β) zu vergleichen, um die Aufmerksamkeit des Fahrzeugführens zu erkennen.

Ferner ist in bevorzugter Ausgestaltung der Erfindung vorgesehen, dass der Gradient mit wenigstens einem vorgebbaren Grenzwert verglichen wird, wobei in weiterer bevorzugter Ausgestaltung der Erfindung vorgesehen ist, dass eine Häufigkeit einer Überschreitung des wenigstens einen Grenzwertes über eine festlegbare Zeitspanne überwacht wird. Auch hierdurch wird in einfacher Weise die Erkennung des Aufmerksamkeitsgrades, insbesondere auch dessen Veränderung über der Zeit, möglich.

Darüber hinaus ist in bevorzugter Ausgestaltung der Erfindung vorgesehen, dass bei einer vorgebbaren Annäherung an den wenigstens einen Grenzwert, einer Überschreitung des wenigstens einen Grenzwertes und/oder einer vorgebbaren Häufigkeit des Überschreitens des wenigstens einen Grenzwertes wenigstens eine Aktion ausgelöst wird, die insbesondere ein automatischer Lenkeingriff, ein akustischer, optischer und/oder haptischer Hinweis an den Fahrzeugführer ist. Hierdurch wird vorteilhaft möglich, bei erkannter Abnahme des Aufmerksamkeitsgrades des Fahrzeugführers diesen darauf hinzuweisen beziehungsweise beim automatischen Lenkeingriff der Verschlechterung des Aufmerksamkeitsgrades entgegenzuwirken. Insgesamt lässt sich hierdurch eine Unfallwahrscheinlichkeit durch nachlassenden Aufmerksamkeitsgrad des Fahrzeugführers verringern. Gegebenenfalls kann bevorzugt vorgesehen sein, dass in Abhängigkeit eines detektierten Aufmerksamkeitsgrades, insbesondere bei erkannter unterschiedlicher Höhe des Nachlassens des Aufmerksamkeitsgrades, abgestufte Aktionen ausgelöst werden. Hierdurch kann die Intensität der Warnung des Fahrzeugführers auf seinen abnehmenden Aufmerksamkeitsgrad variiert werden, so dass der Fahrzeugführer rechtzeitig Hinweise erhält, beispielsweise die Fahrt für eine Pause oder dergleichen zu unterbrechen.

Ferner ist in bevorzugter Ausgestaltung der Erfindung vorgesehen, dass zur Ermittlung des Lenkwinkels und des Lenkradwinkels eine Winkelstellung und/oder eine Drehzahl des Rotors eines Servomotors einer elektrischen Lenkunterstützung und eine Winkelstellung einer Lenksäule ausgewertet werden. Diese Signale stehen in Fahrzeugen, die eine elektrische Lenkunterstützung (EPS) besitzen, für andere Mess- und Steueraufgaben bereits zur Verfügung, so dass eine Anordnung zusätzlicher Sensoren für die Erfassung und Auswertung des Phasenverlaufes von Lenkwinkel und Lenkradwinkel nicht erforderlich ist.

Bevorzugt ist ferner, dass zur Ermittlung des Phasenverlaufes ein Lenkungs-Drehmoment der elektrischen Lenkunterstützung ausgewertet wird. Hierdurch wird vorteilhaft eine indirekte Nutzung bereits vorhandener Sensoren beziehungsweise der Messwerte möglich. Das dem Lenkungs-Drehmoment entsprechende Signal ist funktional abhängig von der Differenz des Lenkwinkels und des Lenkradwinkels und stellt somit indirekt ein redundantes Signal für die Erkennung des Aufmerksamkeitsgrades zur Verfügung. Somit kann insbesondere anhand nur eines Signals eine Auswertung von Phasenunterschieden erfolgen, wodurch die Genauigkeit und die Durchführbarkeit des Verfahrens wesentlich vereinfacht ist.

Erfindungsgemäß wird die Aufgabe ferner durch eine Vorrichtung mit den in den Ansprüchen 11 und 12 genannten Merkmalen gelöst. Dadurch, dass wenigstens eine das Lenkverhalten des Fahrzeuges detektierende Sensiereinrichtung vorgesehen ist, die mit einer Signalerfassungs- und -auswerteeinheit zusammenwirkt, mittels der in Abhängigkeit eines Phasenverlaufes zwischen einem Lenkwinkel wenigstens eines lenkbaren Rades des Fahrzeuges und einem Lenkradwinkel ein dem Aufmerksamkeitsgrad entsprechendes Signal generierbar ist, wird vorteilhaft möglich, die Vorrichtung in einfacher Weise in ein Fahrzeug zu integrieren. Insbesondere kann die Vorrichtung Bestandteil eines Steuergerätes des Fahrzeuges sein.

Weitere bevorzugte Ausgestaltungen der Erfindung ergeben sich aus den übrigen, in den Unteransprüchen genannten Merkmalen.

Die Erfindung wird nachfolgend in Ausführungsbeispielen anhand der zugehörigen Zeichnungen näher erläutert. Es zeigen:
- Figur 1: schematisch eine Lenkungsanordnung eines Fahrzeuges;

- Figuren 2 und 2a: Kennlinienverläufe eines Lenkwinkels und eines Lenkradwinkels und
- Figur 3: ein Blockschaltbild einer Vorrichtung zur Erkennung des Aufmerksamkeitsgrades.

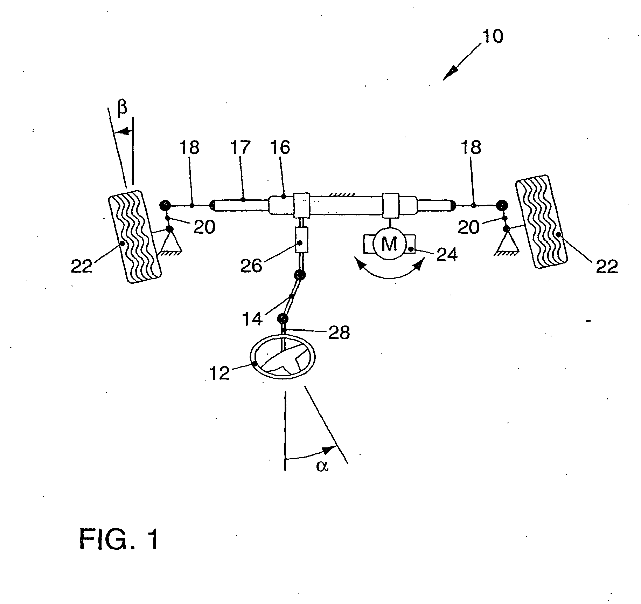

Figur 1 zeigt schematisch eine Lenkung 10 eines Kraftfahrzeuges. Die Lenkung 10 umfasst ein Lenkrad 12, das mit einer Lenkspindel 14 verbunden ist und auf eine in einem Lenkgehäuse 16 angeordnete Zahnstange 17 wirkt. Die Zahnstange 17 ist mit Spurstangen 18 verbunden, die ihrerseits auf Lenkhebel 20 wirken. Die Lenkhebel 20 tragen lenkbare Räder 22 des Kraftfahrzeuges, insbesondere Vorderräder des Kraftfahrzeuges. Der Lenkung 10 ist ein elektrischer Servomotor 24 zur Lenkunterstützung zugeordnet. In die Lenkspindel 14 ist ein Torsionsstab 26 integriert. Ferner ist die Lenkspindel 14 mit einem Lenkwinkelsensor 28 ausgestattet.

Aufbau und Wirkungsweise der schematisch skizzierten Lenkung 10 sind allgemein bekannt, so dass im Rahmen der vorliegenden Beschreibung hierauf nicht näher eingegangen werden soll.

Allgemein ist festzuhalten, dass bei Betätigung der Lenkung 10 über die Wirkverbindung zwischen Lenkrad 12 und lenkbaren Rädern 22 jedem Lenkradwinkel α des Lenkrades 12 ein definierter Lenkwinkel β der Räder 22 zugeordnet ist.

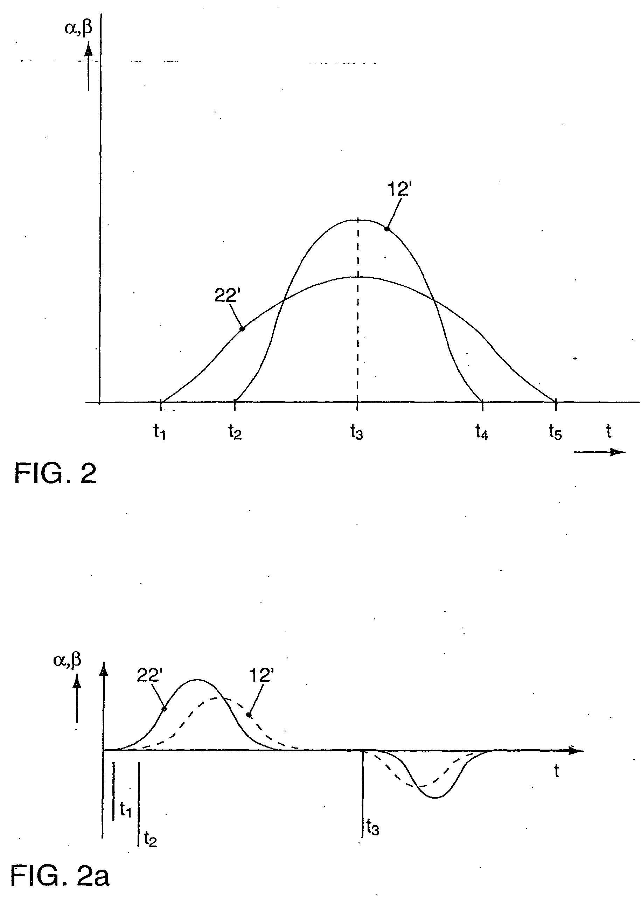

Figur 2 verdeutlicht den Verlauf des Lenkwinkels β als Kennlinie 22' über der Zeit und den Verlauf des Lenkradwinkels α als Kennlinie 12' über der Zeit, wobei hier von dem Ansatz des erfindungsgemäßen Verfahrens ausgegangen wurde. Zunächst ist entscheidend, dass eine Änderung des Lenkwinkels β und des Lenkradwinkels α nicht von einem Fahrzeugführerwunsch ausgeht, sondern durch äußere Einflüsse hervorgerufen ist. Diese können beispielsweise in Windeinflüssen, Hindernissen auf der Straße, schlechten Straßenverhältnissen, schlechtem Geradeauslauf des Fahrzeuges oder dergleichen begründet sein.

Allen diesen äußeren Einflüssen ist gemeinsam, dass zunächst eine Änderung des Lenkwinkels β (Kennlinie 22') eintritt. Figur 2 verdeutlicht, dass zu einem Zeitpunkt t₁ ein äußerer Einfluss gegeben ist, der zu einer Änderung des Lenkwinkels β führt. Konstruktionsbedingt folgt der Änderung des Lenkwinkels β zu einem Zeitpunkt t₂ eine Änderung des Lenkradwinkels α, der hier als Kennlinie 12' eingetragen ist. Die Zeitspanne von t₁ bis t₂ ist durch ein Spiel der Lenkung 10, Ansprechen des Torsionsstabes 26 und dergleichen technisch begründet. Entscheidend ist, dass eine Auswertung im Sinne des erfindungsgemäßen Verfahrens erst dann erfolgt, wenn die Änderung des Lenkwinkels β die Änderung des Lenkradwinkels α bewirkt. Der Fahrzeugführer nimmt die Änderung des Lenkwinkels β um die Zeitspanne t₂ - t₁ verzögert infolge einer Änderung des Lenkradwinkels α wahr. Dies bedeutet: Idealerweise zum Zeitpunkt t₂ nimmt der Fahrzeugführer wahr, dass der Lenkwinkel β, der Ist-Lenkwinkel, vom gewünschten Soll-Lenkwinkel β abweicht. Dies führt zu einer Gegenreaktion des Fahrzeugführers, der den Ist-Lenkwinkel β wiederum auf den Soll-Lenkwinkel β regeln will. Bei einer angenommenen Geradeausfahrt beträgt der Soll-Lenkwinkel β 0° und der Soll-Lenkradwinkel α ebenfalls 0°. Äußere Einflüsse führen dazu, dass zum Zeitpunkt t₁ der Ist-Lenkwinkel β größer wird und mit der Zeitverzögerung t₂ - t₁ der Ist-Lenkradwinkel α ebenfalls größer wird.

Zu einem Zeitpunkt t₃ wird der Fahrzeugführer feststellen, dass der Ist-Lenkwinkel β vom Soll-Lenkwinkel β abweicht und wird durch Betätigung des Lenkrades 12 einen Ausgleich herbeiführen. Dies bedeutet, ab dem Zeitpunkt t₃ wird durch Betätigung des Fahrzeugführers der Lenkradwinkel α verkleinert, dem durch systembedingte Zeitverzögerung der Lenkwinkel β folgt. Demzufolge entspricht der Lenkradwinkel α zum Zeitpunkt t₄ wiederum dem Soll-Lenkradwinkel α, während zum Zeitpunkt t₅ der Ist-Lenkwinkel β dem Soll-Lenkwinkel β entspricht.

Dies zugrunde legend folgt, dass aus dem Verlauf der Zeitspanne t₃ - t₂ auf den Aufmerksamkeitsgrad des Fahrzeugführers geschlossen werden kann. Je aufmerksamer der Fahrzeugführer ist, umso eher wird er feststellen, dass der Ist-Lenkwinkel β vom Soll-Lenkwinkel β abweicht. Dies bedeutet, je kürzer die Zeitspanne t₃ - t₂ ist, umso höher ist der Aufmerksamkeitsgrad des Fahrzeugführers.

Figur 2a zeigt ein Ausführungsbeispiel, bei dem eine Veränderung des Lenkwinkels β durch kurze und impulsartige Störeinflüsse stattfindet. Dies bedeutet, eine Korrektur des Lenkradwinkels α erfolgt erst deutlich nach der kurzen und impulsartigen Störung. Die Reaktion des Fahrers zum Zeitpunkt t₃ erfolgt somit erst, wenn die kurze, impulsartige Störung bereits vorbei ist. Da jedoch auch derartige kurze, impulsartige Störungen zu einer Beeinträchtigung der Geradeausfahrt des Fahrzeuges führen, ist eine Reaktion des Fahrzeugführers unabdingbar. Insofern ist auch hier die Zeitspanne t₃ - t₂ ein Anhaltspunkt für den Aufmerksamkeitsgrad des Fahrzeugführers.

Gemäß einer Variante, die nicht zur Erfindung gehört, wird die Zeitspanne t₃ - t₂ gemessen und mit einem Grenzwert verglichen. Überschreitet die Zeitspanne t₃ - t₂ diesen Grenzwert, wird ein Warnhinweis, der einem Aufmerksamkeitsgrad des Fahrzeugführers entspricht, gegeben. Dieser kann beispielsweise aus einer akustischen, optischen oder haptischen Information bestehen. Überschreitet die Zeitspanne t₃ - t₂ den Grenzwert um einen ebenfalls vorgebbaren Betrag, kann zusätzlich ein automatischer Lenkeingriff erfolgen, der dazu führt, dass der Ist-Lenkwinkel β auf den Soll-Lenkwinkel β zurückgeführt wird.

Gemäß der Erfindung sind folgende varianten möglich :

Eine Auswertevariante besteht darin, dass der Gradient des Anstieges des Lonkradwinkels α beginnend mit dem Zeitpunkt t₂ ausgewertet wird. Ist der Gradient des Lenkradwinkels α größer als ein vorgebbarer Gradient, ohne dass eine Gegenreaktion (Zeitpunkt t₃) des Fahrzeugführers eintritt, kann ebenfalls ein Warnhinweis hinsichtlich eines geminderten Aufmerksamkeitsgrades des Fahrzeugführers erfolgen. Ab einem bestimmten Gradienten, das heißt, ein vorgebbarer Grenzwert wurde überschritten, kann ebenfalls ein automatischer Lenkeingriff erfolgen.

Ein weiterer Ansatz besteht darin, dass der Gradient des Lenkwinkels β ab dem Zeitpunkt t₁ ins Verhältnis gesetzt wird zum Gradienten des Lenkradwinkels α ab dem Zeitpunk t₂ Resultierend aus der Tatsache, dass jedem definierten Lenkwinkel β ein definierter Lenkradwinkel α zugeordnet ist, kann durch Feststellen eines Abfalls des Gradienten des Lenkradwinkels α erkannt werden, dass der Fahrzeugführer eine Abweichung des Ist-Lenkwinkels β vom Soll-Lenkwinkel β erkannt hat und entsprechende Gegenreaktionen ausgelöst hat. Somit ist auch dies ein Indiz für den Aufmerksamkeitsgrad des Fahrzeugführers.

Eine bevorzugte Weiterbildung besteht darin, dass die Häufigkeit als Bezugsgröße herangezogen wird, mit der die Zeitspanne t₃ bis t₂ einen vorgebbaren Grenzwert überschreitet. Die Zeitspanne t₃ bis t₂ definiert letztendlich den Reaktionszeitraum des Fahrzeugführers auf eine äußere Lenkbeeinflussung. Wird die Häufigkeit, mit der die Zeitspanne t₃ bis t₂ einen Grenzwert überschreitet - gegebenenfalls auch geringfügig - größer, ist dies ein weiteres Indiz für einen abnehmenden Aufmerksamkeitsgrad des Fahrzeugführers.

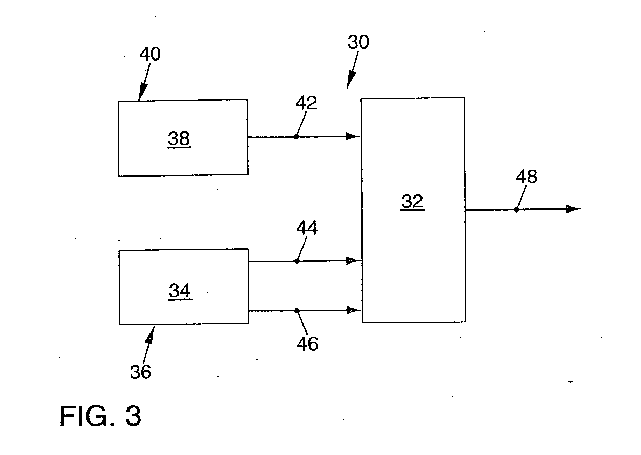

Figur 3 zeigt in einem Blockschaltbild eine Vorrichtung 30 zur Erkennung des Aufmerksamkeitsgrades eines Fahrzeugführers. Die Vorrichtung 30 umfasst eine Signalerfassungs- und -auswerteeinheit 32, die mit einer Steuerung 34 einer elektrischen Lenkunterstützung (EPS) 36 verbunden ist. Ferner kann die Signalerfassungs- und -auswerteeinheit 32 mit einer Steuerung 38 eines elektronischen Stabilitätssystems (ESP) 40 verbunden sein. Der Signalerfassungs- und -auswerteeinheit 32 steht hierbei über das ESP-System beispielsweise ein Signal 42 zur Verfügung, das dem aktuellen Lenkwinkel β entspricht. Über das EPS-System 36 steht der Signalerfassungs- und -auswerteeinheit 32 ein Signal 44 zur Verfügung, das einer Winkelstellung und/oder einer Drehzahl eines Rotors des elektrischen Servomotors 24 entspricht. Ferner steht ein Signal 46 zur Verfügung, das einem aktuellen Lenkungs-Drehmoment, gemessen über den Torsionsstab 26, entspricht. Somit kann die Signalerfassungs- und -auswerteeinheit 32 bei mit dem EPS-System 36 beziehungsweise dem ESP-System 40 ausgestatteten Fahrzeugen auf vorhandene Signale zurückgreifen. Somit sind für die erfindungsgemäße Erkennung des Aufmerksamkeitsgrades des Fahrzeugführers keine zusätzlichen Sensiereinrichtungen oder dergleichen erforderlich. Die Signalerfassungs- und -auswerteeinheit 32 generiert ein Signal 48, das dem Aufmerksamkeitsgrad des Fahrzeugführers entspricht und mittels dem eine entsprechende Aktion, beispielsweise ein Lenkeingriff oder ein akustischer, visueller oder haptischer Warnhinweis erfolgen kann. Entsprechend einem über die Auswertung festgestellten Niveau des Aufmerksamkeitsgrades des Fahrzeugführers, kann mittels des Signals 48 auch eine gestufte Aktion ausgelöst werden. Diese besteht beispielsweise in einem zunächst nachfolgenden optischen Warnhinweis, nachfolgendem kombinierten optischen und akustischen Warnhinweis und gegebenenfalls nachfolgendem optischen akustischen und haptischen Warnhinweis. Schließlich kann bei besonders erheblicher Beeinträchtigung des Aufmerksamkeitsgrades des Fahrzeugführers ein automatischer Lenkeingriff erfolgen.

### BEZUGSZEICHENLISTE

- 10: Lenkung
- 12: Lenkrad
- 12': Kennlinie (Verlauf Lenkradwinkel α)
- 14: Lenkspindel
- 16: Lenkgehäuse
- 17: Zahnstange
- 18: Spurstangen
- 20: Lenkhebel
- 22: Räder
- 22': Kennlinie (Verlauf Lenkwinkel β)
- 24: Servomotor
- 26: Torsionsstab
- 28: Lenkwinkelsensor
- 30: Vorrichtung zur Erkennung des Aufmerksamkeitsgrades
- 32: Signalerfassungs- und -auswerteeinheit
- 34: Steuerung für 36
- 36: elektrische Lenkunterstützung (EPS-System)
- 38: Steuerung für 40
- 40: elektronisches Stabilitätssystem (ESP-System)
- 42: Signal (entspricht Lenkwinkel β)
- 44: Signal (entspricht Winkelstellung und/oder Drehzahl eines Rotors des elektrischen Servomotors 24)
- 46: Signal (entspricht aktuellem Lenkungs-Drehmoment, gemessen über den Torsionsstab 26)
- 48: Signal (entspricht Aufmerksamkeitsgrad des Fahrzeugführers)

- α: Lenkradwinkel
- β: Lenkwinkel
- tₙ: Zeitpunkte

## Patentansprüche

1. Verfahren zur Erkennung des Aufmerksamkeitsgrades eines Fahrzeugführers, wobei das Lenkverhalten des Fahrzeugführers überwacht und ein Phasenverlauf zwischen einer Änderung eines Lenkwinkels (β) wenigstens eines lenkbaren Rades (22) des Fahrzeuges und einer Änderung des Lenkradwinkels (α) ausgewertet wird, sowie ein Zeitpunkt t₂ bestimmt wird, zu dem eine Änderung des Lenkradwinkels (α), folgend einer zu einem Zeitpunkt t₁ beginnenden Änderung des Lenkwinkels (β), einsetzt, ein darauffolgender Zeitpunkt t₃ bestimmt wird ab dem der Fahrzeugführer durch Betätigung des Lenkrades beginnt, einen Ausgleich herbeizuführen und die Erkennung des Aufmerksamkeitsgrades des Fahrzeugführers durch Auswertung des Gradienten des Lenkradwinkels (α) in der Zeitspanne (t₂ bis t₃) erfolgt.

2. Verfahren zur Erkennung des Aufmerksamkeitsgrades eines Fahrzeugführers, wobei das Lenkverhalten des Fahrzeugführers überwacht und ein Phasenverlauf zwischen einer Änderung eines Lenkwinkels (β) wenigstens eines lenkbaren Rades (22) des Fahrzeuges und einer Änderung des Lenkradwinkels (α) ausgewertet wird, sowie ein Zeitpunkt t₂ bestimmt wird, zu dem eine Änderung des Lenkradwinkels (α), folgend einer zu einem Zeitpunkt t₁ beginnenden Änderung des Lenkwinkels (β), einsetzt, ein darauffolgender Zeitpunkt t₃ bestimmt wird, ab dem der Fahrzeugführer durch Betätigung des Lenkrades beginnt, einen Ausgleich herbeizuführen und zur Erkennung des Aufmerksamkeitsgrades des Fahrzeugführers der Gradient des Lenkradwinkels (α) mit einem Gradienten des Lenkwinkels (β) verglichen wird.

3. Verfahren nach Anspruch 1, **dadurch gekennzeichnet, dass** der Gradient des Lenkradwinkels (α) mit wenigstens einem vorgebbaren Grenzwert verglichen wird.

4. Verfahren nach einem der vorhergehenden Ansprüche, **dadurch gekennzeichnet, dass** eine Häufigkeit einer Überschreitung des wenigstens einen Grenzwertes über eine festlegbare Zeitspanne überwacht wird.

5. Verfahren nach einem der vorhergehenden Ansprüche, **dadurch gekennzeichnet, dass** bei einer vorgebbaren Annäherung an den wenigstens einen Grenzwert, einer Überschreitung des wenigstens einen Grenzwertes und/oder einer Häufigkeit des Überschreitens des wenigstens einen Grenzwertes wenigstens eine Aktion ausgelöst wird.

6. Verfahren nach Anspruch 5, **dadurch gekennzeichnet, dass** ein automatischer Lenkeingriff erfolgt.

7. Verfahren nach Anspruch 5, **dadurch gekennzeichnet, dass** wenigstens ein akustischer, optischer und/oder haptischer Hinweis erfolgt.

8. Verfahren nach einem der vorhergehenden Ansprüche, **dadurch gekennzeichnet, dass** in Abhängigkeit eines detektierten Aufmerksamkeitsgrades abgestufte Aktionen ausgelöst werden.

9. Verfahren nach einem der vorhergehenden Ansprüche, **dadurch gekennzeichnet, dass** zur Ermittlung des Lenkwinkels (β) und des Lenkradwinkels (α) eine Winkelstellung und/oder eine Drehzahl des Rotors eines Servomotors (24) einer elektrischen Lenkunterstützung (36) und eine Winkelstellung einer Lenksäule ausgewertet werden.

10. Verfahren nach einem der vorhergehenden Ansprüche, **dadurch gekennzeichnet, dass** zur Ermittlung der Differenz des Lenkwinkels (β) und des Lenkradwinkels (α) eln Lenkungs-Drehmoment einer elektrischen Lenkunterstützung (36) ausgewertet wird.

11. Vorrichtung (30) zur Erkennung des Aufmerksamkeitsgrades eines Fahrzeugführers, mit wenigstens einer das Lenkverhalten des Fahrzeuges detektierenden Sensiereinrichtung und mit einer Signalerfassungs- und -auswerteeinheit (32) zur Erfassung des Phasenverlaufs zwischen einer Änderung des Lenkwinkels (β) wenigstens eines lenkbaren Rades (22) des Fahrzeuges und einer Änderung des Lenkradwinkels (α),
mit Mitteln zum Bestimmen eines Zeitpunktes t₂, zu dem eine Änderung des Lenkradwinkels (α), folgend einer zu einem Zeitpunkt t₁ beginnenden Änderung des Lenkwinkels (β), einsetzt und
mit Mitteln zum Bestimmen eines darauffolgenden Zeitpunktes t₃, ab dem der Fahrzeugführer durch Betätigung des Lenkrades beginnt, einen Ausgleich herbeizuführen,
wobei die Signalerfassungs- und -auswerteeinheit (32) Mittel zum Generieren eines dem Aufmerksamkeitsgrad entsprechenden Signals durch Auswertung des Gradienten des Lenkradwinkels (α) in der Zeitspanne (t₂ bis t₃) enthält.

12. Vorrichtung (30) zur Erkennung des Aufmerksamkeitsgrades eines Fahrzeugführers, mit wenigstens einer das Lenkverhalten des Fahrzeuges detektierenden Sensiereinrichtung und mit einer Signalerfassungs- und -auswerteeinheit (32) zur Erfassung des Phasenverlaufs zwischen einer Änderung des Lenkwinkels (β) wenigstens eines lenkbaren Rades (22) des Fahrzeuges und einer Änderung des Lenkradwinkels (α),
mit Mitteln zum Bestimmen eines Zeitpunktes t₂, zu dem eine Änderung des Lenkradwinkels (α), folgend einer zu einem Zeitpunkt t₁ beginnenden Änderung des Lenkwinkels (β), einsetzt und
mit Mitteln zum Bestimmen eines darauffolgenden Zeitpunktes t₃, ab dem der Fahrzeugführer durch Betätigung des Lenkrades beginnt, einen Ausgleich herbeizuführen,
wobei die Signalerfassungs- und -auswerteeinheit (32) Mittel zum Generieren eines dem Aufmerksamkeitsgrad entsprechenden Signals durch Vergleich des Gradienten des Lenkradwinkels (α) mit dem Gradienten des Lenkwinkels (β) enthält.

## Claims

1. Method for detecting the level of awareness of a vehicle driver, the steering behaviour of the vehicle driver being monitored and a phase profile being evaluated between a change in a steering angle (β) of at least one steerable wheel (22) of the vehicle and a change in the steering-wheel angle (α), and an instant t₂ being determined, at which a change in the steering-wheel angle (α) begins, following a change in the steering angle (β) which begins at an instant t₁, and a following instant t₃ being determined, from which the vehicle driver starts to bring about compensation by actuation of the steering wheel and the level of awareness of the vehicle driver is detected by evaluation of the gradient of the steering-wheel angle (α) in the time period (t₂ to t₃).

2. Method for detecting the level of awareness of a vehicle driver, the steering behaviour of the vehicle driver being monitored and a phase profile being evaluated between a change in a steering angle (β) of at least one steerable wheel (22) of the vehicle and a change in the steering-wheel angle (α), and an instant t₂ being determined, at which a change in the steering-wheel angle (α) begins, following a change in the steering angle (β) which begins at an instant t₁, and a following instant t₃ being determined, from which the vehicle driver starts to bring about compensation by actuation of the steering wheel and the gradient of the steering-wheel angle (α) being compared with a gradient of the steering angle (β) in order to detect the level of awareness of the vehicle driver.

3. Method according to Claim 1, **characterized in that** the gradient of the steering-wheel angle (α) is compared with at least one predefinable limiting value.

4. Method according to one of the preceding claims, **characterized in that** a frequency of exceeding of the at least one limiting value is monitored over a predefinable time period.

5. Method according to one of the preceding claims, **characterized in that** at least one action is triggered at a predefinable approximation to the at least one limiting value, at an exceeding of the at least one limiting value and/or at a frequency of exceeding of the at least one limiting value.

6. Method according to Claim 5, **characterized in that** an automatic steering intervention takes place.

7. Method according to Claim 5, **characterized in that** at least one acoustic, visual and/or haptic indication takes place.

8. Method according to one of the preceding claims, **characterized in that** actions which are graded as a function of a detected level of awareness are triggered.

9. Method according to one of the preceding claims, **characterized in that**, in order to determine the steering angle (β) and the steering-wheel angle (α), an angular position and/or a rotational speed of the rotor of a servomotor (24) of an electric steering assistance means (36) and an angular position of a steering column are evaluated.

10. Method according to one of the preceding claims, **characterized in that**, in order to determine the difference of the steering angle (β) and the steering-wheel angle (α), a steering torque of an electric steering assistance means (36) is evaluated.

11. Apparatus (30) for detecting the level of awareness of a vehicle driver, having at least one sensing device which detects the steering behaviour of the vehicle, and having a signal sensing and evaluation unit (32) for detecting the phase profile between a change in the steering angle (β) of at least one steerable wheel (22) of the vehicle and a change in the steering-wheel angle (α), having means for determining an instant t₂, at which a change in the steering-wheel angle (α) begins, following a change in the steering angle (β) which begins at an instant t₁, and having means for determining a following instant t₃, from which the vehicle driver starts to bring about compensation by actuation of the steering wheel, the signal sensing and evaluation unit (32) comprising means for generating a signal which corresponds to the level of awareness by evaluation of the gradient of the steering-wheel angle (α) in the time period (t₂ to t₃).

12. Apparatus (30) for detecting the level of awareness of a vehicle driver, having at least one sensing device which detects the steering behaviour of the vehicle, and having a signal sensing and evaluation unit (32) for detecting the phase profile between a change in the steering angle (β) of at least one steerable wheel (22) of the vehicle and a change in the steering-wheel angle (α), having means for determining an instant t₂, at which a change in the steering-wheel angle (α) begins, following a change in the steering angle (β) which begins at an instant t₁, and having means for determining a following instant t₃, from which the vehicle driver starts to bring about compensation by actuation of the steering wheel, the signal sensing and evaluation unit (32) comprising means for generating a signal which corresponds to the level of awareness by comparison of the gradient of the steering-wheel angle (α) with the gradient of the steering angle (β).

## Revendications

1. Procédé d'identification du niveau d'attention d'un conducteur de véhicule, le comportement de conduite du conducteur de véhicule étant surveillé et une courbe de phases entre un changement d'un angle de direction (β) d'au moins une roue directrice (22) du véhicule et un changement de l'angle du volant (α) étant évaluée, et un moment t₂, auquel un changement de l'angle du volant (α) consécutif à un changement de l'angle de direction (β) commençant à un moment t₁ se produit, étant déterminé, un moment t₃ consécutif, à partir duquel par manoeuvre du volant, le conducteur du véhicule commence à procéder à une compensation, étant déterminé et l'identification du niveau d'attention du conducteur du véhicule étant effectuée par évaluation du gradient de l'angle du volant (α) dans la période (t₂ à t₃).

2. Procédé d'identification du niveau d'attention d'un conducteur de véhicule, le comportement de conduite du conducteur de véhicule étant surveillé et une courbe de phases entre un changement d'un angle de direction (β) d'au moins une roue directrice (22) du véhicule et un changement de l'angle du volant (α) étant évaluée, et un moment t₂, auquel un changement de l'angle du volant (α) consécutif à un changement de l'angle de direction (β) commençant à un moment t₁ se produit, étant déterminé, un moment t₃ consécutif, à partir duquel par manoeuvre du volant, le conducteur du véhicule commence à procéder à une compensation, étant déterminé et pour l'identification du niveau d'attention du conducteur du véhicule, le gradient de l'angle du volant (α) étant comparé avec le gradient de l'angle de direction (β).

3. Procédé selon la revendication 1, **caractérisé en ce que** le gradient de l'angle du volant (α) est comparé avec au moins une valeur limite prédéfinissable.

4. Procédé selon l'une quelconque des revendications précédentes, **caractérisé en ce qu'**une fréquence d'un dépassement de la au moins une valeur limite est surveillée sur une période définissable.

5. Procédé selon l'une quelconque des revendications précédentes, **caractérisé en ce que** lors d'une approche prédéfinissable de la au moins une valeur limite, d'un dépassement de la au moins une valeur limite et/ou d'une fréquence de dépassement de la au moins une valeur limite, au moins une action est déclenchée.

6. Procédé selon la revendication 5, **caractérisé en ce qu'**une intervention de conduite automatique est effectuée.

7. Procédé selon la revendication 5, **caractérisé en ce qu'**au moins une information acoustique, optique et/ou haptique est donnée.

8. Procédé selon l'une quelconque des revendications précédentes, **caractérisé en ce que**, en fonction d'un niveau d'attention détecté, des actions échelonnées sont déclenchées.

9. Procédé selon l'une quelconque des revendications précédentes, **caractérisé en ce que**, pour la détermination de l'angle de direction (β) et de l'angle du volant (α), une position angulaire et/ou un régime du rotor d'un servomoteur (24) d'une assistance de direction (36) électrique et une position angulaire d'une colonne de direction sont évalués.

10. Procédé selon l'une quelconque des revendications précédentes, **caractérisé en ce que**, pour la détermination de la différence de l'angle de direction (β) et de l'angle du volant (α), un couple de rotation de direction d'une assistance de direction électrique (36) est évalué.

11. Dispositif (30) d'identification du niveau d'attention d'un conducteur de véhicule, avec au moins un système de capteurs détectant le comportement de conduite du véhicule et avec une unité de détection et d'évaluation de signaux (32), pour détecter la courbe de phases entre un changement de l'angle de direction (β) d'au moins une roue directrice (22) du véhicule et un changement de l'angle du volant (α), avec des moyens pour déterminer un moment t₂, auquel un changement de l'angle du volant (α) consécutif à un changement de l'angle de direction (β) commençant à un moment t₁ se produit, et avec des moyens pour déterminer un moment t₃, consécutif, à partir duquel par manoeuvre du volant, le conducteur du véhicule commence à procéder à une compensation, l'unité de détection et d'évaluation de signaux (32) contenant des moyens pour générer un signal correspondant au niveau d'attention, par évaluation du gradient de l'angle du volant (α), dans la période (t₂ à t₃).

12. Dispositif (30) d'identification du niveau d'attention d'un conducteur de véhicule, avec au moins un système de capteurs détectant le comportement de conduite du véhicule et avec une unité de détection et d'évaluation de signaux (32), pour détecter la courbe de phases entre un changement de l'angle de direction (β) d'au moins une roue directrice (22) du véhicule et un changement de l'angle du volant (α), avec des moyens pour déterminer un moment t₂, auquel un changement de l'angle du volant (α) consécutif à un changement de l'angle de direction (β) commençant à un moment t₁ se produit, et avec des moyens pour déterminer un moment t₃ consécutif, à partir duquel par manoeuvre du volant, le conducteur du véhicule commence à procéder à une compensation, l'unité de détection et d'évaluation de signaux (32) contenant des moyens pour générer un signal correspondant au niveau d'attention, par comparaison du gradient de l'angle du volant (α) avec le gradient de l'angle de direction (β).
